(19) Europäisches Patentamt / European Patent Office / Office européen des brevets

(11) **EP 1 795 176 A2**

(12) **DEMANDE DE BREVET EUROPEEN**

(43) Date de publication:
**13.06.2007 Bulletin 2007/24**

(51) Int Cl.:
**A61K 8/04** *(2006.01)* **A61Q 1/02** *(2006.01)*
**A61Q 19/00** *(2006.01)*

(21) Numéro de dépôt: **06301222.3**

(22) Date de dépôt: **07.12.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **08.12.2005 FR 0553803**

(71) Demandeur: **L'ORÉAL**
**75008 Paris (FR)**

(72) Inventeur: **Kasai, Takehiko**
**131-0034, Tokyo (JP)**

(74) Mandataire: **Le Coupanec, Pascale A.M.P.**
**Nony & Associés,**
**3 rue de Penthièvre**
**75008 Paris (FR)**

(54) **Composition cosmétique foisonnée de faible densité**

(57) La présente invention concerne une composition cosmétique foisonnée destinée au soin et/ou maquillage de matières kératiniques comprenant, dans un milieu physiologiquement acceptable, au moins une phase aqueuse et une charge, ladite composition ayant une densité inférieure ou égale à 0,12 g/cm³.

EP 1 795 176 A2

**Description**

La présente invention se rapporte à une composition foisonnée destinée au soin et/ou au maquillage des matières kératiniques et à son utilisation notamment comme base de fond de teint.

Classiquement, les compositions cosmétiques se présentent le plus souvent sous forme de solutions, de gels, de crèmes plus ou moins fluides ou de poudres libres ou compactes. Or, les utilisateurs de produits de soin de la peau recherchent de plus en plus des produits agréables à utiliser et ayant une texture originale.

Ainsi, il a déjà été proposé d'introduire un gaz, généralement de l'air dans des compositions cosmétiques, pour leur conférer une texture légère et leur donner l'aspect d'une mousse. C'est ce que l'on appelle le foisonnement. Les émulsions foisonnées obtenues sont appréciées pour leur légèreté à l'application.

Ce type de compositions cosmétiques se présentent généralement sous la forme de mousses temporaires produites juste avant utilisation.

Il s'agit soit de produits aérosols distribués à partir d'un récipient pressurisé, à l'aide d'un gaz propulseur et formant ainsi une mousse, soit de compositions distribuées à partir d'un récipient au moyen d'une pompe mécanique reliée à une tête de distribution.

Toutefois, les compositions foisonnées actuellement disponibles ne donnent pas toujours totalement satisfaction d'une part en terme de qualité de foisonnement et d'autre part de pérennité dans le temps du foisonnement. Dans la plupart des cas, l'aspect mousse généralement généré au moment de la distribution s'altère très rapidement en terme de densité. Plus précisément, la mousse à très vite tendance à s'affaisser.

La présente invention vise en particulier à proposer une composition foisonnée présentant une densité améliorée, notamment en terme de stabilité.

Ainsi, selon un de ses aspects, l'invention concerne une composition cosmétique foisonnée destinée au soin et/ou maquillage de matières kératiniques comprenant, dans un milieu physiologiquement acceptable, au moins une phase aqueuse et une charge, ladite composition ayant une densité inférieure ou égale à 0,12 g/cm$^3$.

Au sens de l'invention, un milieu physiologiquement acceptable, est un milieu compatible avec la peau, les yeux et/ou les cheveux et donc utile pour formuler une composition cosmétique et/ou dermatologique.

Selon un autre de ses aspects, l'invention concerne un produit pouvant former une composition foisonnée comprenant :

(a) une composition de base, comprenant, dans un milieu physiologiquement acceptable, au moins une phase aqueuse et une charge,
(b) un dispenseur de composition foisonnée pour délivrer ladite composition de base sous forme d'une composition foisonnée de densité inférieure ou égale à 0,12 g/cm$^3$, ce dispenseur comprenant au moins :

-    un réservoir contenant la composition de base, et
-    une tête de distribution pour délivrer la composition foisonnée.

Selon un autre de ses aspects, l'invention concerne un produit pouvant former une composition foisonnée telle que définie précédemment comprenant :

(a) une composition de base, comprenant, dans un milieu physiologiquement acceptable, au moins une phase aqueuse et une charge,
(b) un dispenseur de composition foisonnée pour délivrer ladite composition de base sous forme d'une composition foisonnée de densité inférieure ou égale à 0,12 g/cm$^3$, ce dispenseur comprenant :

-    un réservoir contenant la composition de base et un gaz propulseur,
-    un mécanisme à activer manuellement comportant une pompe pour produire un volume de composition foisonnée, et
-    une tête de distribution, en communication fluidique avec ledit mécanisme, pour délivrer ladite composition foisonnée.

Selon un autre aspect de l'invention, celle-ci concerne un procédé de soin et/ou de maquillage du corps, en particulier du corps humain, notamment du visage, comprenant l'application sur la surface à traiter et/ou maquiller d'une composition foisonnée telle que définie précédemment.

Par "la composition" ou "les compositions" on entend, au sens de l'invention, la composition cosmétique foisonnée et/ou la composition de base.

La composition foisonnée selon l'invention est avantageuse à plusieurs titres. Elle présente une texture mousse légère, onctueuse et fondante lors de son application sur la peau. Elle s'étale facilement sur la peau et permet d'obtenir un maquillage uniforme de la peau sans laisser de traces visibles. De plus, après l'application sur la peau, le maquillage ou le dépôt obtenu présente un fini poudré, velouté et est confortable à porter, sans effet de dessèchement, de tiraillement ; la peau maquillée ou traitée présente une douceur agréable.

COMPOSITION FOISONNÉE

Les compositions foisonnées selon l'invention sont formées de manière stable sous forme de mousse à l'aide d'une composition de base et d'air ou d'un gaz inerte.

L'air ou le gaz inerte peuvent représenter notamment de 10 à 500 %, de préférence de 20 à 200 %, par exemple de 30 à 100 % du volume de la composition foisonnée.

Ce volume peut être calculé en comparant la densité de la composition de base et de la composition foisonnée.

Outre l'air, les gaz permettant d'obtenir la composition foisonnée sont en particulier des gaz inertes, par exemple de l'azote, du dioxyde de carbone, des oxydes d'azote, des gaz nobles, ou un mélange desdits gaz. Lorsque la composition comprend un composé sensible à l'oxydation, il est préférable d'utiliser un gaz sans oxygène tel que l'azote, ou le dioxyde de carbone.

La composition de base servant à obtenir la composition foisonnée a une composition similaire à la composition foisonnée exceptée sa densité plus élevée dans la mesure où elle est dénuée d'air ou de gaz inerte. La quantité de gaz introduite dans la composition de base contribue à l'ajustement de la densité de la composition foisonnée à la valeur souhaitée, c'est à dire inférieure ou égale à 0,12 $g/cm^3$.

Comme précisé précédemment, la composition foisonnée de l'invention présente une densité inférieure à 0,12 $g/cm^3$. La composition foisonnée de l'invention possède avantageusement une densité allant de 0,02 à 0,11 $g/cm^3$ et de préférence de 0,06 à 0,10 $g/cm^3$, cette densité étant mesurée à une température d'environ 20 °C et à la pression atmosphérique selon le protocole suivant.

Le test est réalisé sur un volume de composition de 25 ml introduit dans un godet de plexiglas® poli de 25 ml ($V_1$) définissant un espace de remplissage cylindrique d'une hauteur de 15 mm et d'une base de diamètre de 46 mm. Le godet a une paroi de fond de 10 mm d'épaisseur et une paroi latéral de 12 mm d'épaisseur.

Préalablement à la mesure, la composition à caractériser et le godet sont maintenus à une température de l'ordre de 20 °C. Le godet est taré et la valeur de poids relevée ($M_1$). La composition foisonnée est ensuite introduite dans le godet de manière à en occuper le volume total et en évitant lors du remplissage du godet la formation de bulles d'air. L'ensemble est laissé reposer 10 secondes pour permettre à la mousse de s'expanser totalement. Le haut du godet est ensuite érasé avant sa pesée ($M_2$). La densité est appréciée selon la convention $\rho = (M_2-M_1)/25$.

La composition foisonnée selon l'invention présente une stabilité satisfaisante qui peut être calculée en mesurant un volume de mousse ($V_2$) restant dans le godet au bout de dix minutes selon le protocole décrit précédemment pour la mesure de la densité.

Le rapport $V_2/V_1$ correspond au rapport entre le volume de la composition foisonnée au bout de dix minutes et le volume de la composition foisonnée au bout de dix secondes.

L'expression "stabilité satisfaisante" s'applique notamment aux compositions foisonnées présentant un rapport

$$\frac{V_2}{V_1}$$ supérieur à 0,85, notamment supérieur à 0,90, par exemple supérieur à 0,95.

Pour un poids de composition foisonnée donnée, le volume de la composition foisonnée est inversement proportionnel à la densité de la composition foisonnée. Ainsi, le rapport entre la densité de la composition foisonnée mesurée au bout de dix secondes et la densité de la composition foisonnée mesurée au bout de dix minutes, peut être supérieur à 0,85, notamment supérieur à 0,90, par exemple supérieur à 0,95.

Au sein de la composition foisonnée selon l'invention, les pores d'air peuvent posséder avantageusement une taille moyenne en nombre allant de 20 $\mu$m à 500 $\mu$m, et de préférence allant de 100 $\mu$m à 300 $\mu$m.

PHASE AQUEUSE

La composition selon l'invention comprend généralement une phase aqueuse comprenant de l'eau et éventuellement un solvant organique hydrophile, comme par exemple les monoalcools et les polyols, en particulier les polyols en $C_2$ à $C_4$, par exemple l'éthylèneglycol, le diéthylèneglycol, le butylèneglycol ou le glycérol.

Les solvants sont bien entendu choisi en fonction de leur compatibilité avec la texture mousse recherchée.

La composition selon l'invention peut être exempte de glycérol afin d'éviter d'induire une sensation de collant au toucher.

La composition comprend avantageusement de l'eau. La composition peut comprendre notamment de 35 à 95 %, voire de 50 à 85 % d'eau, par exemple de 60 à 75 % d'eau par rapport à son poids total.

La composition selon l'invention peut comprendre également de l'eau de mer, notamment au moins 2 % en poids, par exemple au moins 5 % en poids d'eau de mer par rapport à leur poids total.

CHARGES

La composition selon l'invention comprend au moins une charge.

Par charges, il faut comprendre des particules de toute forme, incolores ou blanches, minérales ou de synthèse, insolubles dans le milieu des compositions quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®), les poudres de poly-β-alanine, les poudres de polyéthylène, les poudres de polymères de tétrafluoroéthylène (Téflon®), la lauroyl-lysine, l'amidon, le nitrure de bore, les poudres de dioxyde de titane micronisées, les microsphères creuses polymériques telles que celles de chlorure de polyvinylidène/acrylonitrile comme l'Expancel® (Nobel Industrie), de copolymères d'acide acrylique, les poudres de résine de silicone, notamment les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; et leurs mélanges.

Avantageusement, les charges sont choisies parmi les charges de faible densité. Par "faible densité", on entend par exemple des charges ayant une densité inférieure à 3,5 g/cm$^3$, notamment inférieure à 3 g/cm$^3$. Sans vouloir se lier à une théorie quelconque, il semble que les compositions comprenant des charges ayant une gravité trop élevée forment des mousses à porosité plus élevée.

Les compositions peuvent comprendre des charges de faible densité en une teneur allant de 0,5 % à 30 % en poids, par rapport au poids total de la composition, et de préférence allant de 1 % à 15 % en poids, et préférentiellement allant de 1 % à 10 % en poids.

Les compositions peuvent aussi comprendre des charges ayant un indice de réfraction faible et/ou n'absorbant sensiblement pas l'eau et/ou les huiles de la composition.

Un indice de réfraction faible est par exemple un indice de réfraction inférieur à 1,7, par exemple inférieur à 1,6. Une charge n'absorbant sensiblement pas l'eau et/ou les huiles de la composition peut avantageusement contribuer à procurer un aspect transparent à la composition.

Les charges ayant un indice de réfraction faible et/ou n'absorbant sensiblement pas l'eau et/ou les huiles de la composition peuvent être présentes dans la composition en une teneur allant de 0,1 % à 30 % en poids, par rapport au poids total de la composition, et de préférence allant de 1 % à 15 % en poids, et préférentiellement allant de 1 % à 10 % en poids.

Parmi les charges possédant un indice de réfraction inférieur à 1,6, n'absorbant pas sensiblement l'eau et les huiles de la composition et ayant une densité inférieure à 3 g/cm$^3$, on peut par exemple citer le fluorophlogopite, et ses mélanges.

Quelles qu'elles soient, les charges peuvent être présentes dans les compositions en une teneur allant de 0,1 % à 35 % en poids, par rapport au poids total de la composition, de préférence allant de 0,5 % à 30 % en poids, et préférentiellement allant de 1 % à 25 % en poids.

Avantageusement, les charges considérées selon l'invention peuvent être traitées en surface de manière à améliorer leur dispersibilité au sein de la composition foisonnée. De tels traitements relèvent des connaissances de l'homme de l'art et certaines charges ainsi traitées sont en outre disponibles commercialement. On peut par exemple citer la poudre de dioxyde de titane micronisée (15 nm) et traitée en surface par un mélange silice/hydroxyde d'aluminium/ acide alginique, commercialisée sous le dénomination MT-100AQ.

Bien entendu, les compositions selon l'invention peuvent contenir des charges de densité ou d'indice de réfraction élevé ou absorbant l'eau et/ou l'huile de la composition mais en une quantité ajustée de manière à obtenir une mousse ayant une densité, et en particulier une faible porosité et une transparence satisfaisante.

Les compositions selon l'invention peuvent ainsi comprendre moins de 3 %, voire moins de 1,5 % de sulfate de baryum.

Avantageusement, les compositions selon l'invention peuvent comprendre une poudre de polyuréthane.

Plus particulièrement, la poudre de polyuréthane est une poudre de copolymère de diisocyanate d'hexaméthylène et de triméthylol hexyl lactone. Une telle poudre de polyuréthane est notamment vendue sous les dénominations "PLASTIC POWDER D-400", "PLASTIC POWDER D-800" par la société TOSHIKI.

Comme autre poudre de polyuréthane, on peut utiliser celle vendue sous la dénomination "PLASTIC POWDER CS-400" par la société TOSHIKI.

La poudre de polyuréthane peut être présente en une teneur allant de 0,3 % à 5 % en poids, par rapport au poids total de la composition, et de préférence allant de 0,4 % à 4 % en poids, et préférentiellement allant de 0,5 % à 4 % en poids.

La composition de l'invention peut en outre contenir au moins un tensioactif anionique.

Il s'agit de préférence d'au moins un tensioactif anionique moussant choisi parmi les sulfates, les éthers sulfates et leurs sels. Parmi les sels de sulfates et d'éther sulfates, on choisit préférentiellement les sels de sodium et de triéthanolamine. Comme tensioactif anionique moussant, on peut ainsi utiliser le lauryléthersulfate de sodium et notamment ceux commercialisés sous les dénominations TEXAPON par la société Henkel.

De manière générale, la composition de l'invention contient une quantité de tensioactif anionique allant de 0,5 à 50 % en poids et de préférence de 2 à 30 % en poids par rapport au poids total de la composition.

La composition selon l'invention peut en outre contenir un émulsionnant qui peut être choisi parmi tous les émulsionnants classiquement utilisés pour les émulsions H/E.

Comme émulsionnants, on peut citer par exemple :

(1) les tensioactifs non ioniques ayant un HLB supérieur ou égal à 9, tels que les esters d'acide gras et de glycérol oxyéthylénés ; les esters d'acide gras et de sorbitan oxyéthylénés ; les dérivés d'acide gras oxyéthylénés ; les esters d'acide gras et de sucre et notamment les esters gras de sucrose tels que le stéarate de sucrose comme le produit commercialisé sous la dénomination TEGOSOFT PSE 14 1 G par la société Goldschmidt ; les éthers d'alkyl-polyglucoside, et leurs mélanges ;

(2) les émulsionnants siliconés tels que les polydiméthylméthylsiloxanes oxyéthylénés (diméthicone copoyol) comme par exemple celui vendu sous la dénomination "DC2-5695" par la société Dow Corning.

La composition selon l'invention peut comporter par exemple de 0,5 à 50 %, de préférence de 2 à 15 % et mieux de 4 à 10 % en poids d'émulsionnant(s) par rapport au poids total de la composition.

La composition selon l'invention comprend généralement un corps gras solide ou liquide et notamment un corps gras liquide.

La composition selon l'invention peut ainsi comprendre une phase huileuse comprenant au moins une huile pouvant être choisie parmi les huiles volatiles, les huiles non volatiles, et leurs mélanges. Avantageusement, la composition comprend au moins une huile volatile et au moins une huile non volatile.

La composition selon l'invention peut comprendre au moins une huile volatile.

Par "huile volatile", on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 5 centistokes ($5 \times 10^{-6}$ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme huile volatile, on peut utiliser aussi une huile volatile fluorée, on peut citer le nonafluoroéthoxybutane, le nonafluorométhoxybutane, le décafluoropentane, le tétradécafluorohexane, le dodécafluoropentane, et leurs mélanges.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa ( 0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des

**5**

atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam®, le squalane, les huiles de paraffine, et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

La composition selon l'invention comporte avantageusement de 1 à 40 % en poids, de préférence de 2 à 30 % en poids et mieux de 5 à 20 % en poids de phase huileuse par rapport à son poids total.

Les autres corps gras susceptibles d'être présents dans la phase huileuse peuvent être par exemple les acides gras, les alcools gras tels que l'alcool cétylique, et les cires.

Ainsi, la phase grasse peut également comprendre des composés solides à température ambiante (20 °C) tels que des cires, ces composants pouvant améliorer la stabilité de la composition foisonnée. Ces composés sont soit ajoutés sous forme fondue, ou bien sous forme solide dans la phase huileuse, chauffés à une température supérieure à la température de fusion du solide. Ces composés peuvent être des cires ou des composés similaires à des cires, par exemple des cires renouvelables naturelles (cires d'insecte, d'animaux et de plantes), des cires fossiles (cire de pétrole, cire de lignite, cire de tourbe ou ozokérite), les cires synthétiques (Fischer-Tropsch, les cires de polyéthylène ou d'amide), les paraffines à haut point de fusion, les asters, les graisses, les acides carboxyliques à longue chaîne, ou les alcools à chaîne longue en $C_{10}$ à $C_{22}$, chacune de ces cires ayant un point de fusion ou de solidification supérieur à la température ambiante (20 °C).

A titre représentatif et non limitatif des compositions de base convenant à l'invention, on peut plus particulièrement citer celles associant à titre de tensioactifs un cétyl alcool, un cétéaryl alcool et/ou des PEG-10-diméthicone et au moins un composé pulvérulent non hydrosoluble à l'image d'une poudre de polyuréthane comme par exemple la Plastic Powder D-800 ou CS-400® ou du $TiO_2$ traité en surface comme par exemple le produit commercial MT-100AQ.

La composition selon l'invention peut comprendre au moins une matière colorante, notamment choisie parmi les pigments, les nacres, les colorants liposolubles et hydrosolubles, et leurs mélanges.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Par "colorants", il faut comprendre des composés généralement organiques solubles dans l'eau ou dans les corps gras comme les huiles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les matières colorantes peuvent être présentes en une teneur allant de 0,1 % à 30 % en poids, par rapport au poids total de la composition, de préférence allant de 0,1 % à 20 % en poids, de préférence allant de 0,5 % à 15 % en poids, préférentiellement allant de 1 % à 15 % en poids, et plus préférentiellement allant de 5 % à 15 % en poids.

De façon connue, la composition de l'invention peut contenir également des adjuvants habituels dans le domaine cosmétique, tels que les humectants, les conservateurs, les antioxydants, les agents complexants, les solvants, les parfums, les filtres, les bactéricides, les absorbeurs d'odeur, les filtres solaires, les vitamines, les hydratants, les composés auto-bronzants, les actifs antirides. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans des vésicules lipidiques.

La composition foisonnée selon l'invention trouve son application dans un grand nombre de traitements, notamment cosmétiques, de la peau, des lèvres et des cheveux, y compris le cuir chevelu, notamment pour le traitement, la protection, le soin de la peau, des lèvres et/ou des cheveux, et/ou pour le maquillage de la peau et/ou des lèvres. Elle peut être destinée aussi au traitement des peaux sèches et/ou des lèvres sèches.

Aussi, l'invention a encore pour objet l'utilisation cosmétique de la composition foisonnée telle que définie ci-dessus pour le traitement, la protection, le soin de la peau, des lèvres et/ou des cheveux, et/ou pour le maquillage de la peau et/ou des lèvres, en particulier pour la réalisation d'un fond de teint ou une base de fond de teint.

L'invention a également pour objet l'utilisation d'un produit tel que défini précédemment pour former une composition foisonnée servant de base pour la réalisation d'un maquillage de type fond de teint.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition foisonnée peut être obtenue à partir d'une composition de base, c'est-à-dire formée des mêmes ingrédients que ceux présents dans la composition foisonnée, mais en revanche dénuée d'air ou de gaz.

Par exemple, la composition de base peut être placée dans un batteur et battue avec du gaz. La mousse prend alors forme et il est possible d'ajuster sa densité. La composition foisonnée sous forme de mousse peut alors être stockée dans un récipient.

Alternativement ou en complément, la composition foisonnée peut être obtenue à partir d'une composition de base dans un distributeur de compositions foisonnées. Ce distributeur peut être un aérosol, contenant, outre la composition de base, un gaz propulseur.

Ce gaz propulseur peut représenter moins de 20 % de la composition de base, en particulier représenter de 1 à 10 % du poids total de la composition de base. Le gaz propulseur pouvant être utilisé peut être choisi parmi le dioxyde de carbone, l'azote, l'oxyde nitro, les hydrocarbones volatiles tels que le butane, l'isobutane, l'hétobutane, le propane, l'éthane, le pentane, l'isododécane, l'isohexadécane, et leurs mélanges.

La composition de base peut également ne pas contenir de gaz propulseur. Dans ce cas, la composition de base peut se trouver dans un distributeur de compositions foisonnées comprenant une tête de distribution pour délivrer la composition foisonnée, une pompe et un tube plongeur pour transférer la composition du récipient dans la tête pour délivrer le produit. La composition foisonnée est formée en forçant la composition de base à passer au travers d'un matériau comprenant une substance poreuse tel qu'un matériau fritté, une grille filtrante en plastique ou en métal, ou des structures similaires.

De tels dispenseurs sont bien connus de l'homme de l'art et sont décrit dans les brevets U.S. Pat No. 3,709,437 (Wright), U.S. Pat. No. 3,937,364 (Wright), U.S. Pat No. 4,022,351 (Wright), U.S. Pat No. 4,1147,306 (Bennett), U.S.

Pat No. 4,184,615 (Wright), U.S. Pat No. 4,598,862 (Rice), U.S. Pat No. 4,615,467 (Grogan et al.), and U.S. Pat No. 5,364,031 (Tamiguchi et al.).

L'exemple ci-après est donné à titre illustratif et sans caractère limitatif.

## EXEMPLE 1

La composition de base ci-après est une base de fond de teint. Les indices annexés à chacun des composés précisent leur dénomination commerciale respective.

| Phase | Nom INCI | Concentration (% massique) |
|---|---|---|
| A | Polymère réticulé de HDI/trimethylol hexyllactone (et) silice [1] | 0,950 |
| | Carbonate de calcium [2] | 3,012 |
| | Dioxyde de titane (et) silice (et) hydroxyde d'aluminium (et) acide alginique [3] | 2,375 |
| | Fluorphlogopite synthétique (et) huile d'oeuf hydrogénée (et) méthicone [4] | 7,534 |
| | Oxydes de fer (et) phosphate de fluoroalcool en $C_9$-$C_{15}$[5] | 0,133 |
| | Oxydes de fer (et) phosphate de fluoroalcool en $C_9$-$C_{15}$[6] | 0,038 |
| | Oxydes de fer (et) phosphate de fluoroalcool en $C_9$-$C_{15}$[7] | 0,029 |
| | | |
| B | Steareth-10[8] | 0,399 |
| | Chlorphenesine | 0,238 |
| | Methylparaben | 0,285 |
| | Cetearyl alcool (et) polysorbate 60 [9] | 0,665 |
| | Methoxycinnamate d'ethylhexyle [10] | 4,940 |
| | Cetyl alcool [11] | 0,143 |
| | Caprylyl glycol [12] | 0,285 |
| | | |
| C | Eau | 64,872 |
| | Eau de mer | 5,700 |
| | Grycyrrhizate de dipotassium | 0,190 |
| | | |
| D | Propane | 1,160 |
| | Butane | 2,670 |
| | Isobutane | 1,120 |
| | Ethane | 0,005 |
| | Pentane | 0,045 |

**8**

(suite)

| Phase | Nom INCI | Concentration (% massique) |
|---|---|---|
| E | PEG-10 diméthicone [13] | 0,694 |
| | Butylène glycol [14] | 1,900 |
| | phénoxyéthanol | 0,238 |
| | Acétate de tocophéryle [15] | 0,048 |
| | Polysorbate 20 | 0,209 |
| | Parfum [16] | 0,019 |
| | Propylène glycol (et) hexylene glycol (et) extrait d'hamamelis virginiana [17] | 0,095 |

[1] : Plastic Powder D 400
[2] : Socal 90 A
[3] : MT-100 A Q
[4] : SNI - SYNTHETIC MICA PDM - 8 W
[5] : PF5 YELLOW 601
[6] : PF5 RED R516L
[7] : PF5 BLACK BL100
[8] : BRIJ 76
[9] : POLAWAX NF
[10] : UVINUL MC 80
[11] : LANETTE 16
[12] : Dermosoft Octiol
[13] : SH 3746 OIL
[14] : 1,3-Butylene glycol
[15] : DL Alpha Tocopheryl acétate
[16] : LIPS - 1086C
[17] : Hamalelis Essence sur support 36 HMW 1090

Avec un dispositif adapté, il est possible de former à l'aide de cette composition de base, une composition foisonnée ayant une densité de 0,0868 g/cm$^3$ et présentant une stabilité satisfaisante.

**Revendications**

1. Composition cosmétique foisonnée destinée au soin et/ou maquillage de matières kératiniques comprenant, dans un milieu physiologiquement acceptable, au moins une phase aqueuse et une charge, ladite composition ayant une densité inférieure ou égale à 0,12 g/cm$^3$.

2. Composition selon la revendication précédente, ayant une densité allant de 0,02 à 0,11 g/cm$^3$.

3. Composition selon la revendication précédente, ayant une densité allant de 0,06 à 0,10 g/cm$^3$.

4. Composition selon l'une quelconque des revendications précédentes, destinée à l'application sur la peau, notamment la peau humaine, en particulier la peau de visage humain.

5. Composition selon la revendication précédente, sous la forme d'un fond de teint ou d'une base de fond de teint.

6. Composition selon l'une quelconque des revendications précédentes, comprenant de 35 à 95 % en poids d'eau par rapport à son poids total.

7. Composition selon l'une quelconque des revendications précédentes, comprenant au moins un tensio-actif.

8. Composition selon la revendication précédente, comprenant 2 à 50 % en poids de tensio-actif(s) par rapport à son poids total.

**9.** Composition selon la revendication 7 ou 8, dans laquelle le tensio-actif est choisi parmi les alkylpolyglucosides, les esters de maltose, les alcools gras polyglycérolés, les dérivés de glucamine, les carboxylates, les dérivés des aminoacides, les alkyl sulfates, les alkyl éther sulfates, les sulfonates, les iséthionates, les taurates, les sulfosuccinates, les alkyl sutfoacétates, les phosphates et alkylphosphates, les polypeptides, les dérivés anioniques d'alkyl polyglucoside, les savons d'acides gras, les bétaïnes, les N-alkylamidobétaïnes et leurs dérivés, les dérivés de la glycine, les sultaïnes, les alkyl polyaminocarboxylates, les alkylamphoacétates et leurs mélanges.

**10.** Composition selon l'une quelconque des revendications 7 à 9, dans laquelle le tensioactif est un tensioactif moussant.

**11.** Composition selon l'une quelconque des revendications précédentes, comprenant, en outre, au moins un solvant choisi parmi les monoalcools, les polyols et leurs mélanges.

**12.** Composition selon l'une quelconque des revendications précédentes, comprenant de 0,1 à 35 % en poids de charge (s) par rapport à son poids total.

**13.** Composition selon l'une quelconque des revendications précédentes, dans laquelle la charge est choisie parmi le talc, le mica, la silice, le kaolin, les poudres de polyamide, les poudres de poly-$\beta$-alanine, les poudres de polyéthylène, les poudres de polymères de tétrafluoroéthylène, la lauroyl-lysine, l'amidon, le nitrure de bore, les poudres de dioxyde de titane micronisées, les microsphères creuses polymériques telles que celles de chlorure de polyvinylidène/acrylonitrile, de copolymères d'acide acrylique, les poudres de résine de silicone, notamment les poudres de silsesquioxane, les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, et leurs mélanges.

**14.** Composition selon l'une quelconque des revendications précédentes, comprenant au moins une charge ayant une densité inférieure à 3,0 g/cm$^3$.

**15.** Composition selon l'une quelconque des revendications précédentes, comprenant au moins une charge comprenant un indice de réfraction inférieur à 1,60.

**16.** Composition selon l'une quelconque des revendications 1 à 15, comprenant à titre de charge au moins du fluorophlogopite.

**17.** Composition selon l'une quelconque des revendications précédentes, comprenant au moins une poudre de polyuréthane.

**18.** Composition selon l'une quelconque des revendications précédentes, comprenant une huile volatile hydrocarbonée.

**19.** Distributeur pouvant former une composition foisonnée comprenant :

(a) une composition de base, comprenant, dans un milieu physiologiquement acceptable, au moins une phase aqueuse et une charge,
(b) un dispenseur de composition foisonnée pour délivrer ladite composition de base sous forme d'une composition foisonnée de densité inférieure ou égale à 0,12 g/cm$^3$, ce dispenseur comprenant au moins :

- un réservoir contenant la composition de base, et
- une tête de distribution, pour délivrer la composition foisonnée.

**20.** Distributeur pouvant former une composition foisonnée comprenant :

(a) une composition de base, comprenant, dans un milieu physiologiquement acceptable, au moins une phase aqueuse et une charge,
(b) un dispenseur de composition foisonnée pour délivrer ladite composition de base sous forme d'une composition foisonnée de densité inférieure ou égale à 0,12 g/cm$^3$, ce dispenseur comprenant :

- un réservoir contenant la composition de base et un gaz propulseur,
- un mécanisme à activer manuellement comportant une pompe pour produire un volume de composition foisonnée,
- une tête de distribution, en communication fluidique avec ledit mécanisme, pour délivrer ladite composition foisonnée.

21. Distributeur selon la revendication précédente, **caractérisé en ce que** le gaz propulseur est choisi parmi le propane, le butane, l'isobutane, l'éthane, le pentane, l'isododécane, l'isodécane, l'isohexadécane et leurs mélanges.

22. Distributeur selon les revendications 19 à 21, dans lequel ladite composition foisonnée est telle que définie en revendications 1 à 18.

23. Procédé de soin et/ou de maquillage du corps, en particulier du corps humain, notamment du visage, comprenant :

   - l'application sur la surface à traiter et/ou à maquiller d'une composition foisonnée telle que définie en revendications 1 à 18.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 293795 A
- US 3709437 A, Wright
- US 3937364 A, Wright
- US 4022351 A, Wright
- US 41147306 B, Bennett
- US 4184615 A, Wright
- US 4598862 A, Rice
- US 4615467 A, Grogan
- US 5364031 A, Tamiguchi